# EUROPEAN PATENT APPLICATION

(11) **EP 1 944 367 A1**
(43) Date of publication of application: **16.07.2008**
(21) Application number: 06812037.7
(22) Date of filing: 19.10.2006
(51) Int. Cl.: C12N 15/09, C12Q 1/37, G01N 21/64, G01N 21/78, G01N 33/483, G01N 33/68

(54) **METHOD OF MEASURING POLYMERIZATION DEGREE OF PROTEIN**

(30) Priority: 19.10.2005 JP 2005304604
(71) Applicant: Olympus Corporation, Tokyo 151-0072 (JP)
(72) Inventor: NAKATA, Hidetaka, Shibuya-ku, Tokyo 1510072 (JP)
(74) Representative: Wibbelmann, Jobst
(86) International application number: PCT/JP2006/320859
(87) International publication number: WO 2007/046472

(57) **Abstract**

The present invention has an object of providing a method for measuring the degree of polymerization in a protein using a very small sample amount, this method being simple and fast, and able to measure the degree of polymerization in a protein in a form which is closer to its *in vivo* structure than is permitted by conventional methods. The present invention is a method for measuring the degree of polymerization in a protein comprising the steps of: synthesizing a protein so that one molecule of subunit is labeled with one molecule of a fluorescent dye for all of subunits included in the protein; dissociating the protein to at least the subunit level; measuring the fluorescence intensity of the fluorescent dye within a small area before and after the dissociation; and determining the degree of polymerization in the protein based on the information relating to the fluorescence intensity obtained from the measurement.

SEQUENCE LISTING

<110> Olympus Corporation

<120> Method for determining the degree of polymerization in

protein

<130> PC-10361

<160> 1

<210> 1

<211> 13

<212> PRT

<213> Artificial Sequence

<220>

<223> A tag sequence for labeling the N terminus of a target

protein with a fluorescence label. Using this tag sequence, an

amino acid labeled with TAMRA can be introduced into the 9th

residue of the protein.

<400> 1

Met Ser Lys Gln Ile Glu Val Asn Xaa Ser Asn Gln Thr

1               5                   10

## Description

### BACKGROUND OF THE INVENTION

### Filed of the Invention

The present invention relates to a method for determining the degree of polymerization in a protein.

### Description of the Related Art

The analysis of the higher order structure of proteins is extremely useful when analyzing the dynamic behavior of proteins and their interactions with other biological molecules in proteomics research, or when elucidating disease and developing therapeutic methods at the molecular level, for example. More specifically, determining whether the protein of interest is a monomer or polymer, and, if a polymer, how it is polymerized, i.e., how many subunits comprise the polymer, is an essential and fundamental aspect of protein structure analysis.

There are a number of methods currently available for analyzing the degree of polymerization in a subject protein, and these methods are being employed in a variety of technical fields.

For example, *Science* discloses a method that employs X-ray structural analysis to analyze the weight of Streptavidin (Science, Vol. 243, pp. 85-88 (1988)). In this article, the target protein is highly purified, crystallized, and then analyzed using an X-ray structure analyzer or the like.

The process of highly purifying and then crystallizing the protein is essential in this method. As a result, this method has the following problems. First, sample preparation requires a long time. Second, an extremely high level of technique is required for crystallization and analysis. Further, there are those proteins subject to analysis that cannot be crystallized. Accordingly, this conventional method cannot be employed, which is a very great problem. Moreover, in order to crystallize the protein of interest, a large amount of protein is required as a sample.

An additional example is disclosed in *Structure* (Structure, Vol. 16, pp. 223-231, (1989)). This article discloses the structure analysis of a homodimer of calcyclin using NMR. More specifically, the subject protein is expressed in the presence of isotopes, and these isotopes are incorporated into the protein. NMR is then used to analyze the protein structure.

In this method, it is necessary to highly purify the isotope-labeled subject protein. In order to carry out sufficient purification for this type of NMR analysis, the interactions between the subunits contained in the polymer are exposed to conditions that differ from the *in vivo* environment. As a result, it is not possible to determine the size of a targeted protein involved in a reaction in solution, while maintaining its activity that would be obtained *in vivo.* Further, a high degree of specialized knowledge is required in order to analyze the NMR data obtained in this protein analysis, and the analysis requires a long period of time.

*The EMBO Journal* (The EMBO Journal, Vol. 116, pp. 3198-3206, (1997)) discloses a protein analysis method employing SDS electrophoresis. This reference relates to analysis of the DNA binding mode for transcription factors. In this method, two samples of the subject protein are prepared, one of which has been subjected to a cross-linking reaction and one of which has not. Next, these are subjected to SDS electrophoresis, the results thereof are compared, and a determination is made as to whether or not the subject protein is a polymer.

When SDS electrophoresis is employed in this way to analyze the degree of polymerization in a protein, the degree of polymerization in the subject protein can become problematic. Namely, when the degree of polymerization is too large in a detection using SDS electrophoresis, it becomes difficult to accurately detect the degree of polymerization in the case of hexamers, octamers or higher proteins. Moreover, an antibody for the protein is necessary when using SDS electrophoresis. Accordingly, this method cannot be used to discern polymer formation for unknown proteins or proteins for which an antibody cannot be prepared.

Moreover, it is not possible with any of the conventional methods described above to measure the degree of polymerization while maintaining the activity of a protein that is in its physiologic state, present as a solution. Further, effort and time are required to carry out any of these methods, and a large amount of sample is required for the analysis.
[Non-patent literature 1] Patricia C. Weber, Science, Vol. 243, pp. 85-88, (1989)
[Non-patent literature 2] Mallika Sastry, Structure, Vol. 16, pp. 223-231, (1998)
[Non-patent literature 3] Igor Antoshechkin, The EMBO Journal, Vol. 116, pp. 3198-3206, (1997)

### SUMMARY OF THE INVENTION

The present invention was conceived in view of the above described circumstances and has as its objective the provision of a method for measuring the degree of polymerization in a protein using a very small sample amount, this method being simple and fast, and able to measure the degree of polymerization in a protein in a form which is closer to its *in vivo* structure than is permitted by conventional methods.

A further objective of the present invention is the provision of a method that enables analysis of the degree of polymerization in the protein to be carried out with high throughput under an environment that reflects the dynamic structure *in vivo.*

As the result of extensive research, the present inventors discovered a means to resolve the above goal. Namely, the present invention provides the means as disclosed below:
(1) A method for measuring the degree of polymerization in a protein comprising the steps of:
   synthesizing a protein so that one molecule of subunit is labeled with one molecule of a fluorescent dye for all of the subunits included in the protein;
   dissociating the protein to at least the subunit level;
   measuring the fluorescence intensity of the fluorescent dye within a small area before and after the dissociation; and
   determining the degree of polymerization in the protein based on the information relating to the fluorescence intensity obtained by these measurement.
(2) A method according to (1) above, wherein the synthesizing step is carried out via a protein synthesis based on the mRNA that codes for the protein.
(3) A method according to (1) or (2) above, wherein the step of synthesizing the protein comprises:
   obtaining a modified mRNA by substituting a codon at an arbitrary position on the mRNA with a codon corresponding to the anticodon of a tRNA used for labeling; and
   synthesizing the protein under conditions that enable protein synthesis in a cell-free protein synthesis system that includes the modified mRNA, the tRNA for labeling, and tRNAs corresponding to naturally occurring amino acids.
(4) A method according to any one of (1) through (3) above, further comprising the step of purifying the product following protein synthesis.
(5) A method according to (3) or (4) above, wherein the anticodon consists of 3 or more bases.
(6) A method according to any one of (3) through (5) above, wherein said codon at the arbitrary position is selected from the group consisting of an amber codon, an ochre codon and an opal codon.
(7) A method according to any one of (3) through (6) above, wherein the codon at the arbitrary position includes a first artificial nucleic acid, and the anticodon corresponding to this includes a second artificial nucleic acid.
(8) A method according to any one of (3) through (7) above, wherein the tRNA used for labeling has a fluorescently labeled amino acid at its 3' terminus.
(9) A method according to any one of (1) through (8) above, wherein the information relating to the fluorescence intensity is obtained as a flickering in the fluorescence intensity that is generated during an arbitrary continuous time interval, and the obtained information is analyzed.
(10) A method according to any one of (1) through (9) above, wherein the dissociating step is carried out under suitable conditions using proteolytic enzymes, surfactants, reducing agents, or a combination thereof.
(11) A method according to any one of (1) through (10), wherein the method is carried out using a means selected from the group consisting of fluorescence correlation spectroscopy, fluorescence cross correlation spectroscopy, fluorescence intensity distribution analysis, fluorescence intensity multiple distribution analysis, and fluorescence intensity distribution analysis-polarization.
(12) A method according to any one of (1) through (11), wherein the protein is a homopolymer.

The present invention provides a method for measuring the degree of polymerization in a protein using a very small sample amount, this method being simple and fast, and able to measure the degree of polymerization in protein in a form which is closer to the *in vivo* structure than permitted by conventional methods.

The present invention further provides a method that enables analysis of the degree of polymerization in a protein to be carried out with high throughput under an environment that reflects the *in vivo* dynamic structure.

### BRIEF DESCRIPTION OF THE DRAWINGS

Fig. 1 is a schematic showing the conceptual view of protein synthesis in the first preferred embodiment of the invention.
Fig. 2 is a schematic view showing the protein that is the subject of measurement.
Fig. 3 is a schematic view showing a protein labeled by a conventional labeling means.

### Description of the Symbols

- 1: mRNA
- 2: codon for labeling
- 3,10: tRNA
- 4: anticodon
- 5: fluorescently labeled amino acid
- 6: tRNA
- 7: polypeptide
- 8.9: codon
- 11: amino acid
- 12: subunit
- 13: ribosome
- 20,30: labeled protein
- 21,: 23, 25, 27, 31, 33, 34, 37 subunit
- 22,: 24, 26, 28, 32, 35, 36, 38, 39 fluorescent dye molecule

### DETAILED DESCRIPTION OF THE PREFERRED EMBODIMENTS

The present invention is a method for measuring the degree of polymerization in a protein comprising the steps of:
synthesizing a protein so that one molecule of subunit is labeled with one molecule of a fluorescent dye for all of the subunits included in the protein;
dissociating the protein to at least the subunit level;
measuring the fluorescence intensity of the fluorescent dye within a small area before and after the dissociation; and
determining the degree of polymerization in protein based on the information relating to the fluorescence intensity obtained by the measurements.

In accordance with the present invention, the method for measuring the degree of polymerization in a protein passes through two steps, namely, synthesizing while simultaneously labeling the protein which is be measured, and dissociating the obtained labeled protein and determining the degree of polymerization in the protein based on the label applied to each subunit.

### 1. Protein synthesis and labeling with fluorescent dye

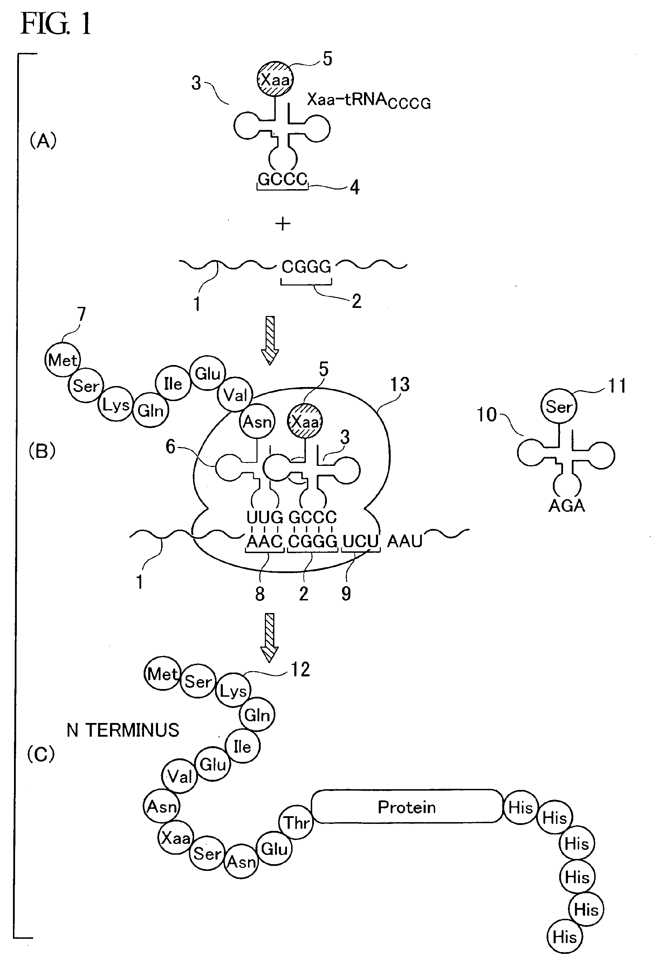

In the method according to the present invention, the protein which is to be measured is synthesized and simultaneously labeled. Namely, labeling using a fluorescent dye is achieved at the time of synthesis of the protein. An overview of the synthesis of a protein according to a first preferred embodiment of the present invention will be explained using FIG. 1.

Referring to FIG. 1(A) modified mRNA1 is prepared in advance in the protein synthesis according to the present invention (FIG. 1(A)). The modified mRNA1 according to the present invention is formed by modifying a portion of the base sequence in the naturally occurring mRNA that codes for the amino acid sequence of the protein which is to be measured. Namely, one codon at the desired position in the base sequence is substituted by labeling codon 2, which corresponds to the anticodon 4 of the tRNA 3 that will be used to carry out fluorescent labeling (FIG. 1(A)). In this example, as shown in FIG. 1(A), anticodon 4 comprises four bases with the amino acid sequence [CCCG]. In addition to anticodon 4, this labeling tRNA 3 has a fluorescently labeled amino acid 5 at its 3' terminus for carrying out the desired labeling. To restate, a labeling anticodon 4 that consists of 4 bases is included at the position where a 3 base anticodon would be present conventionally, and a fluorescently labeled amino acid 5, which is not a naturally occurring amino acid, is present at the 3' end where a naturally occurring amino acid assigned to the base sequence of the anticodon would be present conventionally (Fig. 1(A)).

Referring to Fig. 1(B), protein synthesis according to the present invention is carried out under conditions enabling protein synthesis in the presence of mRNA1, tRNA3 used for labeling, and tRNA corresponding to naturally occurring amino acids that include tRNA6 and 10 (Fig. 1 (B)). Fig. 1 (B) is a view schematically showing part of the protein synthesis carried out under these conditions. In this protein synthesis, polypeptide 7 is formed according to the mRNA1 base sequence. The anticodon 4 of the labeling tRNA3 is bound to codon 2 which is used to accomplish labeling at the desired site. As described above, the labeling tRNA3 has a fluorescently labeled amino acid 5 which is not a naturally occurring amino acid, in place of one of the usual 20 amino acids. On the other hand, tRNAs having naturally occurring amino acids correspond to each of the codons present at positions other than the labeling codon 2 position on mRNA1, as in the case of tRNA10 which has an amino acid 11. Accordingly, as shown in FIG. 1(C), when protein synthesis is complete, a subunit 12 that includes only one molecule of fluorescent dye molecule 5 is obtained (FIG. 1(C)).

When the thus-obtained subunit is present in an aqueous solution at an appropriate concentration and under conditions that approximate *in vivo* conditions, then a suitable degree of polymerization occurs, enabling the target protein to be obtained.

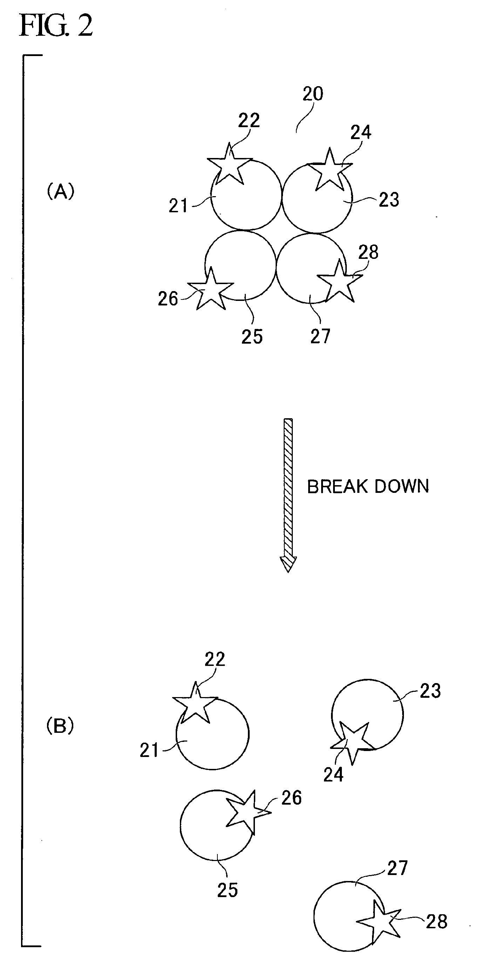

The subject protein obtained according to the present invention is thought to be in the state shown in FIG. 2 (A) when in an aqueous solution approximating *in vivo* conditions. Referring to the schematic view in FIG. 2(A), labeled protein 20, a tetramer, is shown as one example. Each molecule of subunit in the target protein synthesized as described above is labeled with one molecule of fluorescent dye. Namely, subunit 21 has a fluorescent dye molecule 22, subunit 23 has a fluorescent dye molecule 24, subunit 25 has a fluorescent dye molecule 26, and subunit 27 has a fluorescent dye molecule 28 (Fig. 2(A)).

As explained above, the synthesis and fluorescent labeling of the desired protein are achieved simultaneously in the method according to the present invention.

The protein synthesis and labeling using fluorescent dye that are included in the method according to the present invention will be explained further below.

The protein which is the subject of measurement in the present invention may be derived from any type of protein, i.e., naturally occurring and/or synthesized proteins, regardless of the source of its amino acid sequence. Further, the present invention may be directed to proteins that are either monomer or polymer. However, a homopolymer consisting of one type of monomer is the preferred subject of measurement in the present invention.

The term "degree of polymerization" as used in the present invention means the number of subunits forming the target protein, i.e., a number indicating the size of the target protein.

In accordance with the present invention, the aforementioned protein synthesis may be carried out in any type of cell-free system known to one skilled in the art. For example, it is acceptable to employ a cell-free protein synthesis that permits protein synthesis under various conditions derived from, for example, E. coli, wheat germ, insect cell culture, rabbit reticulocyte lysate, etc. In addition to mRNA, such environments may include the necessary elements for protein synthesis such as, for example, ribosomes, amino acids, protein translation factors, ATP, GTP, enzymes, etc. The protein synthesis according to the present invention may employ any type of commercially obtainable kit. These cell-free kits may be obtained from Shimadzu Corp., Toyobo Co., Ltd., Roche, Promega, Marubun Corp. or the like, for example.

The modified mRNA according to the present invention may be obtained by any type of conventionally known method for preparing mRNA. For example, it is acceptable to express the modified mRNA prior to protein synthesis under conditions that enable a conventionally known cell free protein synthesis. In this case, for example, genes that have been made to artificially incorporate a desired partial modification using PCR (polymerase chain reaction) etc. are prepared, and are incorporated into a plasmid vector. The plasmid is then added to the cell-free protein synthesis environment. Such methods as this are conventionally known to one skilled in the art. It is also acceptable in the present invention to formulate the desired modified mRNA using any other method known to one skilled in the art.

The fluorescent dye employed in accordance with the present invention may be any type of conventionally known fluorescent dye that can be linked to the 3' end of the tRNA. Examples of such pigments include TAMRA (carboxymethylrhodamine), TMR (tetramethlrhodamine), Rhodamine Green, Alexa fluor (registered trademark) 488, YOYO1, EVOblue (registered trademark) and Alexa fluor (registered trademark) 647, among others. Note, however, that the fluorescent dye employed in the present invention is not limited to the preceding.

The expression "tRNA used for labeling" which is employed here indicates tRNA that is used for fluorescent labeling of the aforementioned protein. This tRNA may be tRNA that has a fluorescent dye molecule. The preparation of tRNA having a fluorescent dye molecule according to the present invention may be achieved by, for example, synthesizing the tRNA using a means for artificially synthesizing nucleic acids such as PCR, for example, and then linking to the tRNA the amino acids fluorescently labeled using a known nucleic acid modifying method. However, the present invention is not limited thereto; rather any other known method may be employed to prepare tRNA having fluorescently labeled amino acids. The phrase "fluorescently labeled amino acid" as used here means an amino acid to which has been added a single fluorescent dye molecule using any conventionally known method.

As an example of the aqueous solution used in the present invention that approximates *in vivo* conditions, any conventionally known buffer solutions, including phosphate buffer, tris buffer, HEPES buffer or the like, are acceptable.

The preceding embodiment described an example using a 4-base anticodon for the labeling tRNA. However, the labeling tRNA anticodon used in the present invention is not limited thereto. Rather, a codon consisting of three bases, or a codon consisting of five or more bases, may also be used; provided that it is possible to discriminate it from the codons that code for the naturally occurring amino acids. For example, when using a codon consisting of three bases, any stop codon, i.e., amber codon (UAG), ochre codon (i.e., UAA), and opal codon (i.e., UGA) may be employed. It is preferable, however, to use an amber codon consisting of the three-base sequence UAG.

It is also acceptable to use one or more pairs of nucleic acids not corresponding to those present in nature (i.e., artificial nucleic acids) in the aforementioned codon consisting of three, four, or five or more bases. If one such corresponding pair of nucleic acids is arbitrarily designated as X and Y, then the first nucleic acid X is substituted for one base in the desired position on the mRNA and the second nucleic acid Y is included in the anticodon of the tRNA used for labeling. As a result, it is possible to synthesis a protein that includes the subunits that contain the fluorescent dye at the desired position in accordance with the present invention. The term "corresponding" employed here means selective and unique bonding. It is acceptable to employ any conventionally known artificial nucleic acid for the artificial nucleic acid employed in the present invention.

Alternatively, the method disclosed in Japanese Patent Application No. 2005-110595, the Schultz method (Schultz et al (1989) Science, 244, 182-188), and the Chambarlin method (Chambarlin, et al. (1989) J. Am. Chem, Soc., 111, 8013-8014), etc. may be used in the present invention. The aforementioned documents are incorporated by reference in the present specification.

The anticodon of the labeling tRNA that is employed according to the present invention as described above may be an anticodon consisting of three bases or more. However, in order to obtain a sufficient quantity of the target protein, the anticodon of the tRNA that is used to carry out the fluorescent labeling employed in the present invention is preferably an anticodon consisting of three bases or four bases. Moreover, an anticodon consisting of four bases is preferable from the perspective of competition with anticodons having naturally occurring amino acids already assigned to the codon. However, in accordance with the present invention as will be explained below, even in the case where synthesizing the target protein using an anticodon consisting of 3; or 5 or more bases (see the article by Hohsaka, Nucleic Acids Research. 2001, vol. 29, pp. 3646-3651), and/or even if the amount of obtained protein would be too small to permit detection with conventional methods, the quantity is sufficient to permit detection in the present invention (the above article is incorporated in the present specification by reference). Namely, in the case of methods employing X ray, NMR and SDS electrophoresis, X ray and NMR require an amount of protein on the order of *mg,* while SDS electrophoresis requires an amount of protein on the order of µ*g.* However, in the method according to the present invention, provided that the amount of protein is on the order of *nM* (volume=50 µL), then it is possible to measure the degree of polymerization with a high degree of precision. A protein quantity on the nM order is an extremely small amount as compared to the amount of protein required in the conventional technologies discussed above.

### 3. Determining the degree of polymerization in protein

In the method for measuring the degree of polymerization in protein according to the present invention, the degree of polymerization is obtained for the aforementioned protein synthesized as describe above. This principle will be explained using as an example the labeled tetramer protein in FIG. 2.

Referring to Fig. 2, in accordance with the present invention as described above, one fluorescent dye molecule is added to each subunit in the protein to be measured, as shown in FIG. 2(A). If the protein is then dissociated to at least the subunit level and the number of fluorescent dye molecules is detected, it is possible to understand how many subunits are present (Fig. 2(B)). To restate, in Fig. 2(A), the labeled tetramer protein is present as a single molecule. However, by dissociating this structure, the subunits included in the protein are separated and four molecules of subunit are generated (FIG. 2 (B)). Accordingly, by comparing the number of molecules before dissociation as shown in FIG. 2(A) and the number of molecules after breakdown as shown in FIG. 2 (B), it is possible to determine the degree of polymerization in the subject protein.

According to the present invention, the fluorescence intensity of the fluorescent dyes within a small region formed by a confocal optical system may be measured before and after dissociation for the purpose of comparing the number of molecules before and after protein dissociation. For example, the fluorescence correlation spectroscopy method according to Masataka Kinjo may be employed in the present invention (Protein, Nucleic Acid and Enzyme, Vol. 44, 1431-1438 (1999)). Using this method, the fluctuation in fluorescence intensity generated by fluorescent molecules within an arbitrary continuous period of time is measured, and the number of molecules of fluorescent dye present in a small area can be calculated using autocorrelation of the obtained information. The term "arbitrary continuous period of time" as used here is a time period sufficient for permitting measurement of the fluctuation in fluorescence intensity when using fluorescence correlation spectroscopy. The single molecule fluorescence spectroscopy system MF20 (manufactured by Olympus Corp.) may be employed as the device for calculating the number of molecules using the aforementioned fluorescence correlation spectroscopy method.

Any method for dissociating protein known to one skilled in the art may be employed to dissociate protein according to the present invention. While the present invention is not limited to the following, approaches that may be used include denaturation using physical and/or chemical means which will be described below; denaturizing agents such as guanidine hydrochloride and urea; proteolytic enzymes such as protease K, trypsin and papain; surfactants such as SDS, Triton X which includes Triton X (registered trademark) 100 and Tween which includes Tween (registered trademark) 20; reducing agents such as dithiothreitol (DTT), 2-mercaptoethanol or the like. Further, examples of denaturation using physical means according to the present invention include such processes as heating, freezing, melting, ultrasonic, ultraviolet, X-rays or the like. Further, examples of denaturation using chemical means according to the present invention include substances which generate an extreme fluctuation in pH, substances which generate a concentrated buffer, organic solvent, metal salts, surfactants, caotropic agents and the like. Examples of caotropic agents include urea, guanidium salt, and the like. Any of the above-described dissociation means that includes use of the above agents and physical denaturaion may be used alone or in combinations of two or more in accordance with the present invention.

In the present invention, the phrase "dissociation (of a protein) to at least the subunit level" means both the dissociation means to break down the protein to the point where the subunit structure is maintained, and dissociating the protein using decomposition to the subunit structure. As shown in FIG. 2(A), each of the subunits that form the labeled protein 20 in the present invention has a fluorescent dye molecule for each molecule. Accordingly, even if the subunit structure is or is not maintained after dissociation, the degree of polymerization obtained as a result is the same.

In addition, it is acceptable to carry out a simple protein purification prior to obtaining the degree of polymerization in protein synthesized in [1. Protein synthesis and fluorescent dye labeling]. This type of simple purification may be carried out by forming a histidine tag to the C terminus of the protein, recovering it using a nickel column, removing the excess protein in a dilute imidazole buffer, and then washing the column with a highly concentrated imidazole buffer. The purified protein may then be dissolved in a buffer solution such as phosphate buffer (PBS hereinafter), and the degree of polymerization obtained by carrying out a dissociation operation and fluorescence intensity detection.

In addition, it is also acceptable in the present invention to measure the degree of polymerization in the protein of interest using a means other than fluorescence correlation spectroscopy. In other words, the degree of polymerization in a protein may be obtained by calculating the degree of fluorescence intensity generated by the fluorescent dye molecules within a small area created by a confocal optical system, and then using an autocorrelation analysis based on this acquired information to obtain information for the subunits that correspond in a 1:1 ratio with the fluorescent dye molecules, i.e., examples of such information including molecular size, number, brightness, etc. Fluorescence cross correlation spectroscopy, fluorescence intensity distribution analysis, fluorescence intensity multiple distribution analysis and fluorescence intensity distribution analysis-polarization may be used in such measurements. Regardless of the method employed, it is possible to reduce the required sample size to the nM order, which is a characteristic feature of the present invention.

### 4. Comparative Example

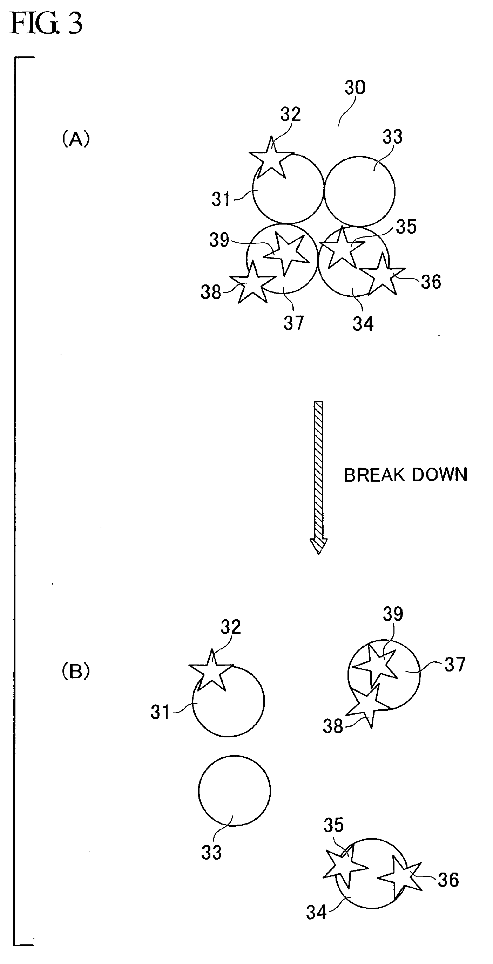

An example employing a means other than that according to the present invention described above is shown in FIG. 3. In this example, a protein is labeled using a conventional labeling means employing a chemical reaction. Referring to FIG. 3, labeled protein 30, a tetramer, which has been fluorescently labeled using a conventional labeling means is shown in FIG. 3 (A). The fluorescent dye molecule added to protein 30 using a chemical reaction is linked to labeled protein 30 unrelated to and irrespective of the presence of the subunits. In the case of this example, a fluorescent dye molecule 32 is linked to subunit 31, no fluorescent dye molecule is linked to subunit 33, two fluorescent dye molecules 35 and 36 are linked to subunit 34, and two fluorescent dye molecules 38 and 39 are linked to subunit 37 (FIG. 3(A)). Accordingly, when the protein is dissociated, despite the fact that four molecules of subunit are present, the information derived based on the fluorescent dye molecules leads to the misinterpretation that there are three subunit molecules present (FIG. 3(B)).

### [Example 1]

The genes for camelid derived single chain antibody (designated "CA" hereinafter), which is a monomer protein, and for streptavidin (designated "SA" hereinafter), which is a tetramer protein, were prepared in advance. These genes were then respectively incorporated into the multicloning site of the plasmid vector pROX-FL (Olympus Corp.) which is used for expressing fluorescently labeled proteins. A portion of this plasmid vector codes for the peptide sequence disclosed in the sequence table. Next, the plasmid was prepared using E. coli DH5α as the host. Using this plasmid, TAMRA-tRNA corresponding to a 4-base codon (Olympus Corp.), and a RTS 100 E. coli HY Kit (Roche), the fluorescent dye TAMRA was labeled to a specific site on the subject protein at a ratio of one molecule per one molecule of subunit. This was then purified via a method employing histidine tags. The labeled proteins were then diluted in solution to achieve a final concentration on the nM order. A protease decomposition reaction was used as the method for dissociating the labeled proteins. Proteinase K (Promega) was used as the protease, the reaction conditions were 37°C for one hour or more at a final concentration of 100 µg/mL, and PBS+0.05% Tween20 was employed as the buffer.

The number of fluorescent dye molecules following sample decomposition was calculated from the fluctuation in the fluorescence generated by the fluorescent molecules within a small area formed by a confocal optical system. The number of fluorescently labeled molecules was calculated by fluorescence intensity distribution analysis (referred to as "FIDA" hereinafter) using the single molecule fluorescence spectroscopy system MF20 manufactured by Olympus Corp. The light source employed was a 543 nm He-Ne laser, and 5 second calculations were carried out 5 times. The results obtained are shown below.

**[Table 1]**

| | Number of fluorescently labeled molecules before dissociation | Number of fluorescently labeled molecules after dissociation | Rate of increase in number of fluorescently labeled molecules before dissociation | Corrected rate of increase | Theoretical rate of increase anticipated based on known reference sources |
|---|---|---|---|---|---|
| CA | 1.73 | 2. 24 | 1. 2 9 | 1. 00 | 1 |
| SA | 1. 48 | 7. 31 1 | 4. 94 | 3. 83 | 4 |

The rate of increase in the number of molecules in the monomer protein CA was 1.29. Accordingly, a corrected value was used in the evaluation that was obtained by dividing the 4.94 obtained for the SA by 1.29. Taking into consideration the possibility that not all of the protein formed tetramers, and that free fluorescent dye may be present, a value of 3.83, versus the theoretical value of 4, was appropriate. The present invention makes it possible to identify the size of the protein formed. Accordingly, it is possible to effectively utilize the present invention's method for measuring the degree of polymerization in protein.

### [Example 2]

The same experiment was performed for calculation employing a fluorescence correlation spectroscopy using the single molecule fluorescence spectroscopy system MF20 manufactured by Olympus Corp., which is capable of calculating the fluctuation in fluorescence generated by fluorescent molecules within a small area formed by a confocal optical system and using autocorrelation of the obtained information to acquire data related to the number of fluorescently labeled molecules. The light source employed was a 543 nm He-Ne laser, and 10 second calculations were carried out 5 times. The results obtained are shown below.

**[Table 2]**

| | Number of fluorescently labeled molecules prior to dissociation | Number of fluorescently labeled molecules after dissociation | Rate of increase in number of fluorescently labeled molecules prior to dissociation | Corrected rate of increase | Theoretical rate of increase anticipated based on known reference sources |
|---|---|---|---|---|---|
| CA | 1.72 | 2.07 | 1.20 | 1.00 | 1 |
| SA | 1.44 | 6.64 | 4.61 | 3.84 | 4 |

The rate of increase in the number of molecules in the monomer protein CA was 1.20. Accordingly, a corrected value was used in the evaluation that was obtained by dividing the 4.61 obtained for the SA by 1.20. Taking into consideration the possibility that not all of the protein formed tetramers, and that free fluorescent dye may be present, a value of 3.84, versus the theoretical value of 4, is appropriate. The present invention makes it possible to identify what mass is formed by the protein. Accordingly, it is possible to effectively utilize the present invention's method for measuring the degree of polymerization in protein.

### [Example 3]

The same operation was carried out as in Example 1, wherein a camelid derived single chain antibody (designated as "CA" hereinafter) and streptavidin (designated as "SA" hereinafter), were labeled with TAMRA. These proteins were then diluted in solution to achieve a final concentration on the nM order. A reaction employing a surfactant agent was used as the method for breaking down the polymer forms. SDS was employed as the surfactant. For the reaction conditions, SDS was added to achieve a final concentration of 0.1%, and incubation for 5 minutes at 95°C was carried out. Calculations were made after compensating the pure water in the evaporated component.

The number of fluorescent molecules in the sample following treatment with the surfactant was calculated using fluorescence intensity distribution analysis using the single molecule fluorescence spectroscopy system MF20 manufactured by Olympus Corp. The light source employed was a 543 nm He-Ne laser, and 5 second calculations were carried out 5 times. The results obtained are shown below.

**[Table 3]**

| | Number of fluorescently labeled molecules prior to dissociation | Number of fluorescently labeled molecules after dissociation | Rate of increase in number of fluorescently labeled molecules prior to dissociation | Corrected rate of increase | Theoretical rate of increase anticipated based on known reference sources |
|---|---|---|---|---|---|
| CA | 4.63 | 5.20 | 1.12 | 1.00 | 1 |
| SA | 0.44 | 1.82 | 4.14 | 3.71 | 4 |

The rate of increase in the monomer protein CA was 1.12. Accordingly, a corrected value was used in the evaluation that was obtained by dividing the 4.14 obtained for the SA by 1.12. Taking into consideration the possibility that not all of the proteins formed tetramers, and that free fluorescent dye may be present, a value of 3.71, versus the theoretical value of 4, was appropriate. The present invention makes it possible to identify the size of the protein formed. Accordingly, it is possible to effectively utilize the present invention's method for measuring the degree of polymerization in protein.

Further effects of the present invention will be explained below.

The method of measurement according to the present invention enables changes in the size or number of fluorescent molecules to be measured with great sensitivity. As a result, the rate of increase from before until after dissociation in the number of fluorescent dye due to breakaway of the fluorescent label during polymer dissociation can be measured with great sensitivity.

By synthesizing a protein fluorescently labeled at a specific position and analyzing this result in the present invention, it is not necessary to provide the protein crystallization step used in X-ray diffraction methods. As a result, it is possible to measure the degree of polymerization in protein quickly and easily. In the present invention, it is possible to synthesize a fluorescently-labeled protein provided that genes thereof can be prepared. Accordingly, it is not necessary to prepare large quantities of protein, or to highly purify it. Moreover, almost all the protein can be expressed and fluorescently labeled in the present invention. As a result, the present invention can be suitably employed for material for which the degree of polymerization in protein cannot be measured because crystallization is not possible. In addition, the measurement of the degree of polymerization in protein is carried out in solution in the present invention. As a result, the fluorescently labeled target protein can be analyzed in a state approximating the *in vivo* condition, in a solution where the protein is reacting or its activity is maintained.

In measuring the degree of polymerization in protein according to the present invention, the analysis of data can be carried out by the extremely simple means of confirming how much the number of fluorescently labeled molecules increases as a result of breaking down the polymers forming the subject protein. Accordingly, the measurements can be carried out within a short period of time without requiring a high degree of specialized knowledge.

Measurement of the degree of polymerization in protein according to the present invention can be carried out under conditions in which other materials or proteins are mixed in the solution. Moreover, the measurements can be carried out easily without requiring the step of highly purifying the protein.

Measurement of the degree of polymerization in protein according to the present invention can be carried out using fluorescently labeled proteins. As a result, it is not necessary to prepare an antibody or the like for the protein, as is required in some conventional methods. Accordingly, it is possible to measure the degree of polymerization in protein even for proteins for which an antibody cannot be prepared. Further, in the measurement of the degree of polymerization in protein according to the first embodiment of the present invention, fluorescently labeled proteins are synthesized from genes, and measurements are then carried out. As a result, it is possible to measure the degree of polymerization in protein even in the case of unknown proteins which previously required purification.

## Claims

1. A method for measuring the degree of polymerization in protein comprising the steps of:
synthesizing a protein so that one molecule of subunit is labeled with one molecule of a fluorescent dye for all of subunits included in said protein;
dissociating said protein to at least the subunit level;
measuring the fluorescence intensity of said fluorescent dye within a small area before and after said dissociation; and
determining the degree of polymerization in said protein based on the information relating to the fluorescence intensity obtained from the measurement.

2. A method according to claim 1, wherein said synthesizing step is carried out via a protein synthesis based on mRNA that codes for said protein.

3. A method according to one of either claim 1 or claim 2, wherein said step of synthesizing the protein comprises:
obtaining a modified mRNA by substituting a codon at an arbitrary position on said mRNA with a codon corresponding to the anticodon of a tRNA used for labeling; and
synthesizing said protein under conditions that enable protein synthesis in a cell-free protein synthesis system that includes said modified mRNA, the tRNA for labeling, and tRNAs corresponding to naturally occurring amino acids.

4. A method according to any one of claims 1 through 3, further comprising the step of purifying the product following said protein synthesis.

5. A method according to claim 3 or 4, wherein said anticodon consists of 3 or more bases.

6. A method according to any one of claims 3 through 5, wherein the codon at the arbitrary position is selected from the group consisting of an amber codon, an ochre codon and an opal codon.

7. A method according to any one of claims 3 through 6, wherein said codon at the arbitrary position includes a first artificial nucleic acid, and said anticodon, corresponding to this includes a second artificial nucleic acid.

8. A method according to any one of claims 3 through 7, wherein said tRNA used for labeling has a fluorescently labeled amino acid at its 3' terminus.

9. A method according to any one of claims 1 through 8, wherein said information relating to the fluorescence intensity is obtained as a flickering in the fluorescence intensity that is generated during an arbitrary continuous time interval, and the obtained information is analyzed.

10. A method according to any one of claims 1 through 9, wherein said dissociating step is carried out under suitable conditions using proteolytic enzymes, surfactants, reducing agents, or a combination thereof.

11. A method according to any one of claims 1 through 10, wherein said method is carried out using a means selected from the group consisting of fluorescence correlation spectroscopy, fluorescence cross correlation spectroscopy, fluorescence intensity distribution analysis, fluorescence intensity multiple distribution analysis, and fluorescence intensity distribution analysis-polarization.

12. A method according to any one of claims 1 through 11, wherein said protein is a homopolymer.
